# EUROPÄISCHE PATENTANMELDUNG

(11) **EP 2 287 334 A1**
(43) Veröffentlichungstag der Anmeldung: **23.02.2011**
(21) Anmeldenummer: 09010757.4
(22) Anmeldetag: 21.08.2009
(51) Int. Cl.: C12Q 1/68

(54) **Verfahren zum Nachweis von Zielnukleinsäuren**

(71) Anmelder: Qiagen GmbH, 40724 Hilden (DE)
(72) Erfinder: Himmelreich, Ralf, Dr., 40764 Langenfeld (DE); Grosshauser, Gerd, 50226 Frechen (DE); Rothmann, Thomas, Dr., 40764 Langenfeld (DE)

(57) **Zusammenfassung**

Die vorliegende Erfindung betrifft ein Verfahren zum Nachweis von Zielnukleinsäuren, wobei die Zielnukleinsäuren mittels eines spezifischen Sequenz-Tags nachgewiesen werden, welcher nicht Teil der Zielnukleinsäure ist.

## Beschreibung

Die vorliegende Erfindung betrifft ein Verfahren zum Nachweis von Zielnukleinsäuren, wobei die Zielnukleinsäuren mittels eines spezifischen Sequenz-Tags nachgewiesen werden, welcher nicht Teil der Zielnukleinsäure ist.

Der Nachweis von Zielnukleinsäuren durch Primer-vermittelte Amplifikation ist in der Molekularbiologie seit vielen Jahren verbreitet. Zu diesen Technologien gehören die Polymerase-Kettenreaktion (PCR), Rolling Circle Amplification (RCA), Strand Displacement Amplification (SDA), Multiple Displacement Amplification (MDA) Strand Displacement Cascade Amplification (SDCA), Self-Sustained Sequence Replication (3SR), Nucleic Acid Based Amplifcation (NASBA), die Amplifikation mit Hilfe der Qβ Replikase sowie weitere lineare Amplifikationstechniken wie zum Beispiel Cylce Sequencing.

Mit Hilfe der PCR und ihren Varianten können bestimmte DNA-Sequenzen, auch aus einem Gemisch verschiedenster Sequenzen, durch Verwenden von sequenzspezifischen Oligonukleotiden, sogenannten Primern, stark vervielfältigt werden. Diese auch als Amplifikation bezeichnete Vervielfältigung erfolgt mit Hilfe einer DNA-Polymerase. DNA-Polymerasen bilden aus einzelsträngiger DNA doppelsträngige DNA, indem sie an freie 3'-OH-Enden eines bereits an den Einzelstrang angelagerten DNA-Fragments die jeweils zum verbleibenden Einzelstrang komplementären Basen anknüpfen. Da DNA-Polymerasen nicht sequenzspezifisch sind, findet ein Auffüllen eines einzelsträngigen Bereiches auf einer DNA-Sequenz immer dann statt, wenn eine partielle Doppelstrangbildung, z.B. durch Anlagerung eines DNA-Fragmentes an den Einezlstrang, vorliegt. Bei der PCR werden zu der DNA, aus der eine bestimmte Sequenz amplifiziert werden soll, sequenzspezifische Primer gegeben, die zu der Sequenz komplementär sind, die vervielfältigt werden soll. Mit Hilfe geeigneter Primer ist es auch möglich, Nukleotidsequenzen in ein Amplifikat einzubauen, die nicht in der ursprünglichen Nukleotidsequenz der Zielnukleinsäure vorhanden waren. Zu diesem Zweck werden am 5'-Ende des Primers Nukleotidsequenzen gewählt, die nicht mit der Zielnukleinsäure komplementär sind. Dadurch ist es beispielsweise möglich, in die Amplifikate einer Zielnukleinsäure Schnittstellen für Restriktionsendonukleasen oder Erkennungssequenzen für andere Nukleinsäure bindende Proteine einzubauen, die in der ursprünglichen Zielnukleinsäure nicht vorhanden gewesen waren.

Die PCR ist eine einfache und flexible Methode zur Vermehrung und zur eingeschränkten Veränderung der Nukleotidsequenz einer Zielnukleinsäure, die mit Abstand am häufigsten zur Amplifikation in der Molekularbiologie verwendet wird. Um die Amplifikation zu erreichen, muss jedoch ein Protokoll durchlaufen werden, das in 20 bis 40 Zyklen mindestens zwei, sehr häufig jedoch drei verschiedene Temperaturschritte durchläuft. Dies erfordert eine spezialisierte Instrumentierung und einen relativ zeitaufwändigen Prozess, da in jedem Zyklus nur eine Verdopplung der Zielnukleinsäure möglich ist.

Die Rolling Circle Amplification (RCA), vermittelt durch eine DNA-Polymerase mit Strang-Verdrängungs-Aktivität und fehlender 5'-3' Exonuklease-Aktivität, kann zirkuläre Oligonukleotide unter isothermalen Bedingungen replizieren. Wenn ein einziger Primer verwendet wird, bildet die RCA innerhalb weniger Minuten eine einzelsträngige lineare Kette von hunderten oder tausenden Tandem-DNA-Kopien einer Zielnukleinsäure, die kovalent mit dieser Zielnukleinsäure verbunden sind. Die Bildung eines linearen Amplifikationsproduktes erlaubt sowohl die räumliche Auflösung als auch die genaue Quantifizierung einer Zielnukleinsäure. Die DNA, die durch die RCA gebildet wird, kann durch fluoreszente Oligonukleotid-Tags markiert werden, die an vielen Stellen in den Tandem-DNA Sequenzen hybridisieren können, da sich die Sequenz der Zielnukleinsäure fortwährend wiederholt. Die RCA kann in Lösung, in situ und in Microarrays durchgeführt werden. In Festphasen-Formaten ist die RCA ausreichend sensitiv, um ein einzelnes Molekül einer Zielnukleinsäure nachweisen zu können.

Eine komplexere Variante der RCA stellt die RCA mit einem Paar unterschiedlicher Primer dar. Diese wird als hyperbranched, ramification oder cascade RCA bezeichnet. Wie in der linearen RCA ist ein Primer komplementär zur Zielnukleinsäure, wohingegen der zweite Primer an die einzelsträngigen DNA-Bereiche binden kann, die durch das primäre RCA Produkt entstanden sind. In der Folge verläuft die RCA in diesem Falle als Kettenreaktion mit einer Kaskade aus einer Vielzahl an Hybridisierungen, Primer-Verlängerungen und Strang-Verdrängungen, an der beide Primer beteiligt sind. Das Ergebnis dieser Reaktion sind konkatemere doppelsträngige DNA Fragmente. Durch diese Art der RCA können innerhalb einer Stunde bis zu etwa 10⁹ Kopien der Zielnukleinsäure hergestellt werden.

Sowohl bei der PCR mit ihren Variationen als auch bei der RCA mit ihren Variationen erfolgt der Nachweis der Zielnukleinsäure über die Amplifikation eines Teils der Zielnukleinsäure.

Eine weitere Methode zum Nachweis von Zielnukleinsäuren stellt die Nicking Endonuclease Signal Amplification (NESA) dar, bei der die Signalamplifikation über kurze Oligonukleotide erfolgt. NESA ist eine sensitive Methode zum Nachweis von spezifischer DNA, bei der das Signal mit Hilfe einer Nicking-Endonuklease amplifiziert wird. Doppelsträngige DNA, die eine Erkennungsstelle für eine Nicking-Endonuklease besitzt, wird denaturiert. Anschließend wird ein im Überschuss vorhandenes Fluoreszenz-markiertes komplementäres Oligonukleotid an diese Zielnukleinsäure hybridisiert, so dass das markierte Oligonukleotid zusammen mit einem Strang der Zielnukleinsäure eine Erkennungsstelle für eine Nicking-Endonuklease enthält. Diese Nicking-Endonuklease schneidet das markierte Oligonukleotid, lässt aber die Zielnukleinsäure intakt. Da die beiden Oligonukleotide durch den Schnitt durch die Endonuklease nun kürzer sind als das ursrüngliche ungeschnittene markierte Oligonukleotid, ist ihre Affinität an die Zielnukleinsäure bei der eingestellten Versuchstemperatur nicht mehr ausreichend, so dass die beiden kurzen Oligonukleotide, von denen eines noch die Fluoreszenzmarkierung besitzt, von der Zielnukleinsäure dissoziieren. Nach der Dissoziation der beiden Oligonukleotide von der Zielnukleinsäure kann die Zielnukleinsäure mit einem weiteren noch ungeschnittenen Fluoreszenz-markierten Oligonukleotid hybridisieren, und ein neuer Zyklus wird eingeleitet. Der Nachweis der Zielnukleinsäure erfolgt über die in diesem Prozess erhaltenen kurzen Fluoreszenz-markierten Oligonukleotide. Zu diesem Zweck wird der Reaktionsansatz nach Abschluss der Reaktion einer Kapillarelektrophorese unterzogen, in der die kürzeren Fluoreszenz markierten Oligonukleotide von den nicht geschnittenen längeren unterschieden werden können.

Im Vergleich zur PCR ist diese Methode zum Nachweis von Zielnukleinsäuren wesentlich schneller, die Reaktionszeit beträgt je nach der Konzentration der Zielnukleinsäure zwischen 10 und 30 min. Dazu müssen jeweils noch etwa 10 min für die Denaturierung der doppelsträngigen Nukleinsäure sowie etwa 10 min für die Kapillarelektrophorese gerechnet werden.

Eine weitere Methode zum Nachweis von Zielnukleinsäuren mit Hilfe einer Schnittstelle für eine Endonuklease stellen die Exponential Amplification Reaction (EXPAR) und ihre lineare Amplifikationsvariante dar. Bei der linearen Variante hybridisiert ein komplementäres Oligonukleotid mit einer Erkennungssequenz für eine Nicking-Endonuklease an eine einzelsträngige Zielnukleinsäure. Nach dem Nicking besteht das Oligonukleotid nunmehr aus zwei kürzeren Oligonukleotiden, die an die Zielnukleinsäure gebunden sind. Die Versuchsbedingungen, die Länge der beiden geschnittenen Oligonukleotide sowie die Reaktionstemperatur, sind so gewählt, dass das kürzere Oligonukleotid von der Zielnukleinsäure dissoziiert, nicht aber das längere. Das längere Oligonukleotid, das an der Zielnukleinsäure verblieben ist, dient nun als Primer, so dass der einzelsträngige Bereich der Zielnukleinsäure wieder aufgefüllt wird. Im nächsten Zyklus schneidet wieder die Nicking-Endonuklease den Einzelstrang, und ein weiteres kurzes Oligonukleotid dissoziiert von der Zielnukleinsäure. Der Nachweis der Zielnukleinsäure erfolgt über die in dieser Reaktion entstandenen kurzen Oligonukleotide über Massenspektrometrie.

Bei der exponentiellen Variante wird das dissoziierende Oligonukleotid der linearen Amplifikation dazu verwendet, einen neuen Primer zu bilden, der an ein so genanntes Amplifikations-Templat binden kann. Dieses Amplifikations-Templat wird zusätzlich zur Zielnukleinsäure zum Reaktionsansatz gegeben. Das Amplifikations-Templat besitzt eine Erkennungsstelle für die Nicking-Endonuklease. Zusätzlich kann sowohl am 5'- als auch am 3'-Ende das dissoziierende Oligonukleotid binden. Im ersten Schritt bindet das im vorhergehenden Zyklus dissoziierte Oligonukleotid an die komplementäre Sequenz, die 5' von der Erkennungsstelle für die Nicking-Endonuklease liegt. Zwar ist dieses Bindeereignis relativ selten, da die Reaktionsbedingungen so gewählt sind, dass das Oligonukleotid eher von seiner komplementären Sequenz dissoziiert als an sie bindet, jedoch reicht diese schwache Bindungsaffinität dennoch aus, um einen transienten Komplex zwischen dem dissoziierten Oligonukleotid und dem Amplifikations-Templat auszubilden. Im zweiten Schritt wird das Oligonukleotid, das nun als Primer dient, an seinem 3' Ende über das gesamte Amplifikations-Templat hinweg verlängert. Es entsteht somit ein doppelsträngiges Amplifikations-Templat, welches eine Erkennungsstelle für die Nicking-Endonuklease besitzt und nach Schneiden durch die Nicking-Endonuklease wiederum ein kurzes dissoziierendes Oligonukleotid freisetzt, welches wiederum an ein weiteres Amplifikations-Templat binden kann. Auch bei diesem Verfahren wird die Detektion des dissoziierten kurzen Oligonukleotids mit Hilfe von Massenspektrometrie durchgeführt.

Sowohl NESA als auch EXPAR nutzen die Eigenschaften einer Nicking-Endonuklease zur Generierung von Oligonukleotiden, die anschließend spezifisch nachgewiesen werden können. Beide Reaktionen haben den Vorteil gegenüber einer PCR, dass sie unter isothermalen Bedingungen ablaufen können und dass die Reaktion sehr schnell vonstatten geht.

Beide Methoden haben aber auch erhebliche Nachteile, die ihre universelle Einsetzbarkeit deutlich einschränken. So sind sowohl NESA als auch EXPAR in der Wahl der möglichen Sequenzen für die nachzuweisenden Zielnukleinsäuren limitiert. Für beide Verfahren ist es zwingend erforderlich, dass bereits auf der Zielnukleinsäure eine Erkennungsstelle für eine Nicking-Endonuklease vorhanden ist. Da es aber nur sehr wenige verschiedene Nicking-Endonukleasen gibt, die unterschiedliche Nukleotidsequenzen erkennen, gibt es nur sehr wenige natürlich vorkommende Nukleinsäuren, die in ihrer Sequenz eine Erkennungsstelle für eine Nicking-Endonuklease beinhalten. Somit ist es nicht möglich, den Großteil der natürlich vorkommenden Nukleinsäuren direkt mit Hilfe von NESA oder EXPAR nachzuweisen. Desweiteren sind auch die Wahl der Versuchsbedingungen sowie die Wahl der Oligonukleotide, die zur spezifischen Detektion verwendet werden können, sehr stark eingeschränkt. Zum einen müssen die beiden Hälften, in die das Oligonukleotid durch die Nicking-Endonuklease geschnitten wird, sich in ihrer Affinität zur Zielnukleinsäure deutlich unterscheiden, damit gewährleistet ist, dass nur die kürzere der beiden Oligonukleotidhälften, nicht aber die längere, von der Zielnukleinsäure dissoziiert. Dies erfordert einen bestimmten engen Temperaturbereich für die Versuchsdurchfühung, der aber nicht zwangsläufig auch kompatibel ist mit den Temperaturoptima der an der Reaktion beteiligten Enzyme. Die Folge davon ist, dass auch unter diesen Gesichtspunkten sowohl NESA als auch EXPAR nicht universell einsetzbar sind.

Zum Anderen ist auch dadurch, dass diese Oligonukleotide vollständige Sequenzkomplementarität zur Zielnukleinsäure aufweisen müssen, ein Multiplex-Ansatz nur sehr eingeschränkt möglich. Zwar lassen sich im Falle von NESA die Oligonukleotide auf verschiedene Weise mit Fluoreszenzfarbstoffen markieren, jedoch gibt es nur eine begrenzte Anzahl an Farbstoffen, die bei verschiedenen Wellenlängen detektiert werden können, so dass die Multiplex-Eigenschaften stark eingeschränkt sind.

Im Fall von EXPAR ist ein Multiplexing nur über die Wahl verschieden langer Oligonukleotide, die von der Zielnukleinsäure dissoziieren, möglich, wodurch die Zahl der parallelen Analysen von Zielnukleinsäuren sehr stark begrenzt ist, zumal auch noch zusätzlich ein Temperaturbereich gefunden werden muss, bei dem von den durch die Nicking-Endonuklease geschnittenen Oligonukleotiden alle kürzeren erhaltenen Oligonukleotidhälften, die alle eine unterschiedliche Länge aufweisen müssen, von der Zielnukleinsäure dissoziieren, die längeren aber auf dieser verbleiben müssen. Soll ein exponentieller Nachweis durch EXPAR möglich sein, müssen zusätzlich noch alle diese kürzeren Oligonukleotidhälften mit gleicher Effizienz einen transienten Komplex mit einem Amplifikations-Templat ausbilden können, um eine Verfälschung der Quantifizierung auszuschließen zu können, was experimentell nur sehr schwer möglich ist. Daher lassen sich sowohl EXPAR als auch NESA in der Praxis kaum für einen Multiplexing-Ansatz verwenden, bei dem parallel mehrere Zielnukleinsäuren gleichzeitig nachgewiesen werden können.

Aufgabe der vorliegenden Erfindung ist es, die aus dem Stand der Technik bekannten Nachteile zu überwinden und ein Verfahren zum Nachweis von Zielnukleinsäuren bereitzustellen, das es ermöglicht, auch mehr als eine Zielnukleinsäure im parallelen Ansatz durch Multiplexing nachzuweisen und bei dem die Nukleotidsequenz der Zielnukleinsäure keine Erkennungssequenz für eine Endonuklease aufweisen muss.

Diese Aufgabe wird gelöst durch ein Verfahren zum Nachweis von Zielnukleinsäuren, umfassend die folgenden Verfahrensschritte:
a) Primer-vermittelte Amplifikation mindestens einer Zielnukleinsäure, wobei mindestens einer der zur Amplifikation verwendeten Primer ein Sequenz-Tag-Primer ist, der an seinem 5'-Ende einen nicht hybridisierenden Teil aufweist, wobei dieser nicht hybridisierende Teil eine erste Sequenz aufweist, die auf dem zu ihm komplementären neu synthetisierten Strang eine Schnittstelle für eine Nicking-Endonuklease während der Amplifikation erzeugt, und des Weiteren 5' von dieser ersten Sequenz eine zweite Sequenz aufweist, die auf dem zu ihm komplementären neu synthetisierten Strang einen Sequenz-Tag während der Amplifikation erzeugt;
b) in Kontakt bringen des zumindest teilweise doppelsträngigen Amplifikats aus Schritt a) mit Nukleotiden, einer Nicking-Endonuklease und einer Polymerase, wobei die Polymerase eine Strang-Verdrängungs-Aktivität und keine 5'→3' Exonuklease-Aktivität besitzt;
c) Isothermale Amplifikation des in Schritt a) erzeugten Sequenz-Tags durch eine oder mehrfache Wiederholung eines Zyklus, aufweisend die folgenden Schritte:
   i) Einfügen eines Nicks an der in Schritt a) eingefügten Schnittstelle mittels der Endonuklease aus Schritt b);
   ii) Auffüllen des Nicks beginnend an dem in Schritt i) erzeugten freien 3'-Ende mit komplementären Nukleotiden mittels der Polymerase aus Schritt b) unter gleichzeitiger Verdrängung des Sequenz-Tags aus dem Doppelstrang.
d) Spezifische Detektion des in Schritt c) amplifizierten Sequenz-Tags.

Mit dem erfindungsgemäßen Verfahren kann eine oder mehr als eine Zielnukleinsäure nachgewiesen werden. Bei der oder den Zielnukleinsäure(n), die mittels des erfindungsgemäßen Verfahrens nachgewiesen werden können, kann es sich um DNA, um RNA oder um ein Gemisch von diesen handeln. In einer bevorzugten Ausführungsform wird DNA nachgewiesen. Bei der DNA kann es sich jeweils um eine oder mehrere cDNAs, genomische DNAs oder ein Fragment von diesen, um Plasmid-DNAs oder ein Fragment von diesen, um virale DNAs oder ein Fragment von diesen, um mitochondriale DNAs oder ein Fragment von diesen, um plastidäre DNAs oder ein Fragment von diesen, oder um die Kombination von zweien oder mehrerer dieser DNAs handeln. Die Zielnukleinsäuren, die durch das erfindungsgemäße Verfahren nachgewiesen werden, müssen keine spezifischen Nukleotidsequenzen aufweisen, die als Erkennungs- und/oder Bindungsstelle für Nicking-Endonukleasen dienen, und benötigen auch darüber hinaus keine spezifischen Sequenz- oder Strukturmotive. Auch Zielnukleinsäuren, die sich nur in einem Nukleotid in ihrer Sequenz unterscheiden, können durch das erfindungsgemäße Verfahren spezifisch nachgewiesen werden. Es kann somit vorteilhafterweise jede spezifische Nukleinsäure unabhängig von ihrer Nukleotidsequenz nachgewiesen werden.

In einem ersten Reaktionsschritt a) wird die Zielnukleinsäure Primer-vermittelt amplifiziert. Dabei ist mindestens einer der verwendeten Primer ein Sequenz-Tag-Primer. Ein Sequenz-Tag-Primer ist **dadurch gekennzeichnet, dass** er an seinem 5'-Ende einen Teil aufweist, der nicht an die Zielnukleinsäure hybridisiert. Dieser nicht hybridisierende Teil weist eine erste Sequenz auf, die auf dem zu ihm komplementären neu synthetisierenden Strang eine Schnittstelle für eine Nicking-Endonuklease während der Amplifikation erzeugt. Des Weiteren weist dieser nicht hybridisierende Teil 5' von dieser ersten Sequenz eine zweite Sequenz auf, die auf dem zu ihm komplementären neu synthetisierten Strang einen Sequenz-Tag während der Amplifikation erzeugt. Die amplifizierte Zielnukleinsäure weist demzufolge nach der Amplifikation einen zumindest teilweise doppelsträngigen Bereich auf, der neben der Sequenz der Zielnukleinsäure auf dem Gegenstrang eine erste Sequenz aufweist, die als Schnittstelle für eine Nicking-Endonuklease dient sowie eine zweite Sequenz, die eine von der Zielnukleinsäure unabhängige Sequenz aufweist und nachfolgend als Sequenz-Tag Verwendung findet. Der Sequenz-Tag ist also eine Sequenz, die komplementär zum 5'-Ende des Sequenz-Tag-Primers - und zwar 5' der Endonuklease-Schnittstelle - ist. Durch die Amplifikation der Zielnukleinsäure in Schritt a) mit den erfindungsgemäßen Primern, von denen mindestens einer ein Sequenz-Tag-Primer ist, weisen die Amplifikate nun in ihrer Nukleotidsequenz zusätzlich zur Nukleotidsequenz eines Teilbereiches oder der gesamten Sequenz der Zielnukleinsäure am 3'- Ende des Gegenstranges zunächst eine Schnittstelle für eine Nicking-Endonuklease auf sowie weiterhin 3' davon einen Sequenz-Tag, der nachfolgend als eine die Zielnukleinsäure charakterisierende Kennzeichnungssequenz verwendet wird. Der Sequenz-Tag wird in Schritt d) des erfindungsgemäßen Verfahrens spezifisch detektiert. Die spezifische Detektion des Sequenz-Tags in Schritt d) bewirkt somit den spezifischen Nachweis der Zielnukleinsäure.

Die Nukleotidabfolge des Sequenz-Tags ist frei wählbar, sie muss im Sinne der vorliegenden Erfindung nur so beschaffen sein, dass sie nicht an die Zielnukleinsäure(n) oder an eine andere Nukleinsäure, die während der Amplifikation im Reaktionsansatz vorhanden ist, hybridisieren kann.

In einer bevorzugten Ausführungsform besitzt der Sequenz-Tag eine Länge von mindestens 12 Nukleotiden, besonders bevorzugt von mindestens 15 Nukleotiden, ganz besonders bevorzugt besitzt der Sequenz-Tag eine Länge von 18 bis 30 Nukleotiden.

In einer weiteren bevorzugten Ausführungsform besitzt der Sequenz-Tag einen GC-Gehalt von 40 bis 60%, weist eine Schmelztemperatur von 48 bis 74 °C auf.

In einer weiteren bevorzugten Ausführungsform besitzt der Sequenz-Tag keine Dimere oder Hairpins ausbildenden Sequenzmotive.

In einer bevorzugten Ausführungsform werden in Schritt a) des erfindungsgemäßen Verfahrens zwei oder mehr Zielnukleinsäuren amplifiziert. Diese Amplifikation kann in demselben Reaktionsgefäß als Multiplex-Amplifikation durchgeführt werden, sie kann aber auch im parallelen Ansatz in räumlich getrennten Reaktionsgefäßen durchgeführt werden. Für jede Zielnukleinsäure wird bevorzugt jeweils mindestens ein spezifischer Sequenz-Tag-Primer zur Amplifikation verwendet.

In einer besonders bevorzugten Ausführungsform ist die Schnittstelle für eine Nicking-Endonuklease für jeden der verwendeten Sequenz-Tag-Primer identisch, so dass im Multiplexing-Ansatz nur eine Spezies Nicking-Endonuklease benötigt wird, die in der Lage ist, einen Nick ausschließlich auf dem Gegenstrang aller in Schritt a) amplifizierten Zielnukleinsäuren zu setzen.

Dahingegen unterscheidet sich für jeden Sequenz-Tag-Primer in einer besonders bevorzugten Ausführungsform die Sequenz des Sequenz-Tags, so dass jede Zielnukleinsäure, die in Schritt a) im Multiplexing-Ansatz oder im parallelen Ansatz amplifiziert wird, durch einen unterschiedlichen Sequenz-Tag gekennzeichnet ist, der als die jeweilige die Zielnukleinsäure charakterisierende Kennzeichnungssequenz verwendet wird. Für jede Zielnukleinsäure, wird für die Amplifikation demnach erfindungsgemäß ein Sequenz-Tag-Primer verwendet, der eine spezifische Sequenz des Sequenz-Tags erzeugt. Die Detektion des Sequenz-Tags kann dabei im parallelen Ansatz in mehr als einem Reaktionsgefäß oder vorteilhafterweise im Multiplexing-Ansatz in einem Reaktionsgefäß erfolgen.

Zur Primer-vermittelten Amplifikation der Zielnukleinsäure eignen sich isothermale Amplifikationsmethoden, die dem Fachmann aus dem Stand der Technik bekannt sind. Zu diesen gehören beispielsweise iCNA (Takara), tHDA (BioHelix) und RPA (TwistDx).

In einer bevorzugten Ausführungsform erfolgt die Primer-vermittelte Amplifikation der Zielnukleinsäure in Schritt a) mittels Polymerasekettenreaktion (PCR). Aus dem Stand der Technik sind diverse Ausführungsformen der PCR bekannt und dem Fachmann geläufig.

In einer besonders bevorzugten Ausführungsform findet die Amplifikation der Zielnukleinsäure mittels PCR mit Hilfe von Nested Primern statt. Bei diesem aus dem Stand der Technik wohlbekannten Verfahren wird zunächst mit einem äußeren Primerpaar ein Teilbereich der Zielnukleinsäure vervielfältigt. Dieser Teilbereich dient anschließend als Templat für eine zweite Amplifikation mit einem zweiten Primerpaar, dessen Primer jeweils 3' vom jeweiligen Primer des ersten Primerpaares binden und somit einen "inneren Bereich" des Templats weiter amplifizieren. Durch diese Art der Amplifikation der Zielnukleinsäure in zwei Schritten wird eine höhere Spezifität der Vervielfältigung erreicht. Bei der PCR mit Hilfe von Nested Primem ist mindestens ein Primer des zweiten inneren Primerpaares ein Sequenz-Tag-Primer.

Das Primer-vermittelte Amplifikat kann nach der Amplifikation vollständig als Doppelstrang vorliegen, aber auch nur teilweise doppelsträngig sein. In diesem Fall weist das Amplifikat neben einem doppelsträngigen Bereich einen oder mehrere weitere Bereiche auf, in denen das Amplifikat als Einzelstrang vorliegt. Im erfindungsgemäßen Verfahren ist es hingegen essentiell, dass der Bereich des Amplifikates, der von der Nicking-Endonuklease erkannt und geschnitten wird, als Doppelstrang vorliegt.

Eine Nicking-Endonuklease erkennt in Schritt b) die Erkennungssequenz auf dem Doppelstrang des Amplifikates und schneidet je nach der Art der Nicking-Endonuklease in oder neben dieser Erkennungssequenz den Gegenstrang zum Sequenz-Tag-Primer, wodurch ein so genannter Nick in das doppelsträngige Amplifikat eingefügt wird, bei dem die 5'-3' Phosphodiester-Bindung zwischen zwei Nukleotiden hydrolytisch gespalten wird. Die Nicking-Endonuklease wirkt also als Phosphodiesterase, so dass in dem Doppelstrang ein Einzelstrangbruch eingefügt wird und ein freies 3'-OH Ende erzeugt wird, welches als Ansatzpunkt für eine Polymerase dient. Im erfindungsgemäßen Verfahren wird immer nur ein Strang des Doppelstranges geschnitten, und zwar der Gegenstrang, auf dem sich auch die Erkennungssequenz für die Nicking-Endonuklease befindet, der andere Strang bleibt intakt. Endonukleasen, die nicht nur einen Strang des Doppelstranges sondern beide schneiden, sind für das erfindungsgemäße Verfahren nicht geeignet.

Geeignete Nicking-Endonukleasen sind beispielsweise Nt.BstNBI, Nt.BspQI, Nb.BbvCI, Nb.BsmI, Nb.BsrDI, Nb.BtsI, Nt.AlwI, Nt.BbvCI, Nt.CviPII, Nt.BsmAI, Nb.Bpu10I und Nt.Bpu10I. Weitere geeignete Nicking-Endonukleasen sind dem Fachmann geläufig.

Der Nick im doppelsträngigen Amplifikat wird von einer Polymerase, die keine 5'→3' Exonukleaseaktivität und weiterhin eine Strang-Verdrängungs-Aktivität besitzt, erkannt. Eine Polymerase im Sinne der vorliegenden Erfindung ist ein Enzym zur Nukleinsäure-Replikation und/oder zur Nukleinsäure-Reparatur. Die Polymerase füllt den Nick am 3'-OH Ende beginnend auf mit Nukleotiden, die komplementär sind zum Templat-Strang. Dazu wird sukzessiv jeweils eines der (Desoxy)-Ribonukleotidphosphate entsprechend der komplementären Base im Matrizenstrang angelagert und unter Abspaltung von Pyrophosphaten über eine Phosphodiester-Bindung eingebaut. Die Polymerisationsreaktion erfolgt immer in 5'→3'-Richtung. Während dieses Prozesses wird durch die Strang-Verdrängungs-Aktivität der Polymerase der Sequenz-Tag von der amplifizierten Nukleinsäure verdrängt und liegt unhybridisiert als Einzelstrang vor.

Geeignete Polymerasen sind alle Polymerasen, die eine Strang-Verdrängungs-Aktivität besitzen und gleichzeitig keine 5'→3' Exonukleaseaktivität.

Bevorzugt handelt es sich bei der Polymerase um eine DNA-Polymerase, die mit Desoxyribonukleotiden den Nick auffüllt. Dazu gehören beispielsweise Vent exo⁻, Deep Vent exo⁻, Bst exo⁻, Klenow-Fragment der DNA-Polymerase I, Phi 29 DNA-Polymerase und 9°Nm DNA-Polymerase. Weitere geeignete DNA-Polymerasen sind dem Fachmann geläufig.

In einer bevorzugten Ausführungsform liegen die Temperaturoptima für die enzymatische Aktivität der Nicking-Endonuklease und der Polymerase in einem ähnlichen Temperaturbereich, eine solche Kombination ist beispielsweise die Kombination der Nicking-Endonuklease Nt.BstNBI mit der DNA-Polymerase Vent exo⁻. Weitere bevorzugte Kombinationen ergeben sich mühelos aus dem Vergleich der Temperaturoptima einer Nicking-Endonuklease mit einer Polymerase. Diese Temperaturoptima sind dem Fachmann ebenfalls geläufig.

Nachdem in Schritt c) des Anspruchs 1 der Nick durch die Polymerase erkannt und mit zum Amplifikat komplementären Nukleotiden aufgefüllt wurde, wobei der Sequenz-Tag vom Amplifikat verdrängt wurde, liegt das Amplifikat nun wieder als in diesem Bereich intakter Doppelstrang vor und besitzt wieder eine Schnittstelle und eine Erkennungssequenz für eine Nicking-Endonuklease sowie einen Sequenz-Tag. Das Amplifikat weist nun also in diesem Bereich wieder dieselbe Nukleinsäuresequenz auf wie vor dem Einführen des Nicks durch die Nicking-Endonuklease, so dass der Vorgang des Einfügens eines Nicks durch die Nicking-Endonuklease, das Auffüllen des Nicks durch die Polymerase sowie das Verdrängen des Sequenz-Tags beliebig oft wiederholt werden kann, so dass der Sequenz-Tag isothermal amplifiziert wird. Durch die Polymerase wird folglich nur die Sequenz, die komplementär zum 5'-Ende des Primers ist, aufgefüllt.

Die Menge des isothermal amplifizierten und freigesetzten Sequenz-Tags aus Schritt c) kann durch mehrere Einflussfaktoren moduliert werden. Einen großen Einfluß auf die erzielte Menge hat die Reaktionszeit, während der die isothermale Amplifikation des Sequenz-Tags mit den Schritten des Anspruchs 1) c) i) und ii) abläuft. Je länger die Reaktionszeit, desto mehr Sequenz-Tags können normalerweise gebildet und freigesetzt werden.

Einen weiteren wichtigen Einflussfaktor stellt die Reaktionstemperatur in Schritt c) dar. Wie bei allen enzymatischen Reaktionen erfolgt die Umsetzung umso schneller, je mehr die Reaktionstemperatur mit dem Temperaturoptimum für die Aktivität der beteiligten Enzyme übereinstimmt. Die isothermale Amplifikation des Sequenz-Tags ist folglich umso effizienter, je ähnlicher die Temperaturoptima der beteiligten Enzyme, der Nicking-Endonuklease und der Polymerase sind, und je genauer die Reaktionstemperatur mit diesen Temperaturoptima übereinstimmt.

Die Wahl der Reaktionstemperatur spielt auch noch in anderer Hinsicht eine wesentliche Rolle für die Amplifikation des Sequenz-Tags. Liegt die Reaktionstemperatur unterhalb der Schmelztemperatur des Sequenz-Tags an das Amplifikat aus Schritt a), so kann der Sequenz-Tag nach Einfügen des Nicks nur durch eine Polymerase mit Strang-Verdrängungs-Aktivität freigesetzt werden. Liegt die Reaktionstemperatur oberhalb der Schmelztemperatur des Sequenz-Tags an das Amplifikat, so ist dieses Hybrid nach Einfügen des Nicks so instabil, dass der Sequenz-Tag auch ohne eine Polymerase mit Strang-Verdrängungs-Aktivität freigesetzt werden kann. In diesem Fall wird der Sequenz-Tag nicht aktiv verdrängt, sondern passiv freigesetzt.

In einer bevorzugten Ausführungsform ist die Reaktionstemperatur in Schritt c) so gewählt, dass der Sequenz-Tag durch die Strang-Verdrängungs-Aktivität einer Polymerase freigesetzt wird, um zu gewährleisten, dass parallel zur Freisetzung des Sequenz-Tags der Nick mit komplementären Nukleotiden wieder aufgefüllt worden ist.

In einer besonders bevorzugten Ausführungsform ist die Reaktionstemperatur in Schritt c) so gewählt, dass der Sequenz-Tag durch die Strang-Verdrängungs-Aktivität einer Polymerase freigesetzt wird und dass die Reaktionstemperatur mit den Temperaturoptima der Nicking-Endonuklease und der Polymerase übereinstimmt.

Weitere Faktoren, die die Menge des Sequenz-Tags beeinflussen, umfassen die Menge an amplifizierter Zielnukleinsäure, die Menge an Nukleotiden sowie die Pufferbedingungen. Weitere Einflussfaktoren sind dem Fachmann geläufig.

In Schritt d) des erfindungsgemäßen Verfahrens werden die in Schritt c) isothermal amplifizierten Sequenz-Tags mit Hilfe einer Sonde, die gegen den Sequenz-Tag gerichtet ist, spezifisch detektiert. Die Sonde muss demzufolge zumindest in einem Teilbereich komplementär zum Sequenz-Tag sein und an diesen hybridisieren können.

Die Sonde ist bevorzugt ein Oligonukleotid, dem Fachmann sind alternative Lösungen geläufig.

Die Sonde kann dabei unterschiedliche Modifikationen aufweisen, wie beispielsweise Fluoreszenzfarbstoffe, Quantum-Dots oder Gold-Nanopartikel. Weitere Möglichkeiten der Modifikation sind dem Fachmann geläufig.

In einer bevorzugten Ausführungsform besitzt die Sonde eine Länge von mindestens 12 Nukleotiden, besonders bevorzugt von mindestens 15 Nukleotiden, ganz besonders bevorzugt besitzt die Sonde eine Länge von 18 Nukleotiden bis 30 Nukleotiden.

In einer weiteren bevorzugten Ausführungsform besitzt die Sonde einen GC-Gehalt von 40 bis 60%, weist eine Schmelztemperatur von 48 bis 74 °C auf.

Möglichkeiten zur Detektion des Hybrids aus Sequenz-Tag und Sonde sind dem Fachmann aus dem Stand der Technik geläufig. In einer Ausführungsform ist die Sonde mit Fluoreszenzfarbstoffen markiert, und die Detektion erfolgt über die Messung der Fluoreszenzänderung, nachdem die Sonde an den Sequenz-Tag gebunden hat.

In einer bevorzugten Ausführungsform gehört die Sonde zur Klasse der Dual-Labeled Probes. Diese sind erfindungsgemäß Oligonukleotide, die sowohl mit einem Reporter-Fluorophor als auch mit einem Quencher gekoppelt sind. Die Nukleotide am 5'-Ende der Sonde sind zu denen am 3'-Ende komplementär, so dass sich eine charakteristische Sekundärstruktur ausbilden kann. Ein Beispiel für solch eine charakteristische Sekundärstruktur ist eine so genannte Haarnadelstruktur, wie sie zum Beispiel in Molecular Beacons zu finden ist. Im Zustand der Haarnadelstruktur emittiert die Sonde durch ihren geringen Abstand zwischen Fluorophor und Quencher keine oder nur sehr wenig Fluoreszenz. Durch die Anlagerung der Sonde an den Sequenz-Tag wird der Abstand zwischen Fluorophor und Quencher vergrößert, wodurch eine Zunahme der Fluoreszenzemission beobachtet werden kann.

Ein weiteres Beispiel für eine Dual-Labeled Probe umfasst erfindungsgemäß eine Hydrolyse-Sonde, beispielsweise eine TaqMan^{®}-Sonde, bei der die Sonde während der Synthese des Gegenstranges am 5'-Ende abgebaut wird und somit Fluorophor und Quencher in einen größeren Abstand zueinander gebracht werden.

Weitere Ausführungsformen von Dual-Labeled Probes sind dem Fachmann geläufig.

In einer weiteren bevorzugten Ausführungsform erfolgt die Detektion des Sequenz-Tags durch eine Sonde mittels Schmelzkurvenanalyse. Bei einer Schmelzkurvenanalyse wird die doppelsträngige Nukleinsäure aufgeschmolzen, indem die Temperatur langsam und kontinuierlich erhöht wird. Bei einer für das Hybrid aus Sonde und Sequenz-Tag spezifischen Schmelztemperatur denaturiert der Doppelstrang zu zwei einzelsträngigen Molekülen. Dadurch, dass einzelsträngige Nukleinsäuren bei 260 nm andere Absorptionseigenschaften besitzen als doppelsträngige, lässt sich eine Schmelzkurvenanalyse bei 260 nm durchrühren, ohne dass Farbstoffmarkierungen an der Sonde oder in doppelsträngige Nukleinsäuren interkalierende Substanzen zur Detektion erforderlich wären.

In einer besonders bevorzugten Ausführungsform erfolgt die Schmelzkurvenanalyse mit Hilfe von für doppelsträngige DNA spezifische Farbstoffe, beispielsweise mit Hilfe von interkalierenden Fluoreszenzfarbstoffen. Bei der Schmelztemperatur denaturiert der Doppelstrang, der aus der Sonde und dem Sequenz-Tag gebildet wird, zu zwei einzelsträngigen Molekülen. Dabei wird der Fluoreszenzfarbstoff freigesetzt, und es wird eine Fluoreszenzabnahme registriert. Beispiele für in doppelsträngige DNA interkalierende Fluoreszenzfarbstoffe sind SYBR^{®} Green und EvaGreen^{®}. Dem Fachmann sind viele weitere Doppelstrang-DNA spezifische Fluoreszenzfarbstoffe bekannt, die für die erfindungsgemäße Schmelzkurvenanalyse geeignet sind.

In einer weiteren besonders bevorzugten Ausführungsform erfolgt die Schmelzkurvenanalyse mit Hilfe einer markierten Sonde. Beispiele für solch eine Markierung sind Fluoreszenzfarbstoffe oder Quantum-Dots. Dem Fachmann sind weitere Möglichkeiten zur Markierung der Sonde geläufig.

In einer bevorzugten Ausführungsform wird mehr als ein Sequenz-Tag in einem Multiplexing-Ansatz detektiert.

In einer bevorzugten Ausführungsform unterscheidet sich die Sequenz des Sequenz-Tags in ihrer Nukleotid-Abfolge in dem Maße, dass im Multiplexing-Ansatz mehrere unterschiedliche Sonden an unterschiedliche Sequenz-Tags binden können und somit mehrere verschiedene Sequenz-Tags gleichzeitig in einem Reaktionsgefäß detektiert werden können.

In einer besonders bevorzugten Ausführungsform unterscheidet sich jeder Sequenz-Tag in seiner Nukleotidsequenz und/oder in seiner Schmelztemperatur mit seiner zugehörigen komplementären Sonde, so dass im Multiplexing-Ansatz in Schritt d) aufgrund unterschiedlicher Schmelztemperaturen mehrere verschiedene Sequenz-Tags gleichzeitig detektiert werden können.

Die Anzahl der in einem Multiplexing-Ansatz parallel analysierbaren Zielnukleinsäuren ergibt sich dabei aus der Anzahl der unterschiedlichen Schmelztemperaturen, die durch das entsprechende Analysegerät voneinander unterschieden werden können kombiniert mit der Anzahl der verschiedenen Fluoreszenzfarbstoffe, die bei unterschiedlichen Wellenlängen vom jeweiligen Analysegerät voneinander unterschieden werden können. Besitzen zwei Sequenz-Tags dieselbe Schmelztemperatur, so können diese dennoch gemeinsam in einem Multiplexing-Ansatz spezifisch detektiert und voneinander unterschieden werden, wenn ihre jeweiligen zugehörigen Sonden unterschiedliche Fluoreszenzfarbstoffe aufweisen, die bei unterschiedlicher Wellenlänge die Fluoreszenz emittieren, so dass diese in unterschiedlichen Fluoreszenzkanälen detektiert werden können. Umgekehrt können Sonden, die mit unterschiedlichen Sequenz-Tags hybridisieren, dieselbe Fluoreszenzmarkierung besitzen, wenn sich die Schmelztemperaturen dieser Sonden mit ihrem zugehörigen Sequenz-Tag unterscheiden. Diese werden dann im selben Fluoreszenzkanal detektiert, können aber durch ihre unterschiedlichen Schmelztemperaturen dennoch voneinander unterschieden werden.

Im Prinzip sind zur Detektion des Sequenz-Tags mittels einer geeigneten Sonde alle Geräte geeignet, die in der Lage sind, im entsprechenden Wellenlängenbereich Änderungen zu detektieren.

Im Falle einer Schmelzkurvenanalyse ohne die Zugabe von Farbstoffen sind hierzu Geräte geeignet, die in der Lage sind, Absorptionsunterschiede in einem Wellenlängenbereich von etwa 260 nm zu messen. Zu diesen Geräten gehören beispielsweise UV-Spectrophotometer. Dem Fachmann sind weitere geeignete Geräte geläufig.

Erfolgt die Detektion des Sequenz-Tags mit Hilfe eines Fluoreszenzfarbstoffes, so sind zur Detektion alle Geräte geeignet, die in der Lage sind, die Fluoreszenz der entsprechenden Wellenlänge zu messen. Zu diesen gehören beispielsweise Real-Time PCR-Cycler oder Spectrophotometer. Weitere geeignete technische Geräte sind dem Fachmann wohlbekannt.

Abbildungen:

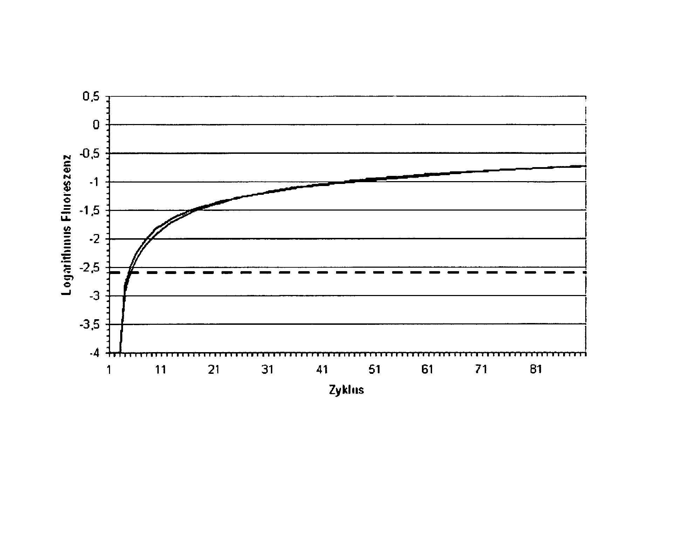

Abbildung 1 zeigt die Fluoreszenzentwicklung der isothermalen Amplifikation in Abhängigkeit von der Zeit. Auf der x-Achse ist die Zahl der Zyklen angegeben, auf der y-Achse der Logarithmus der Fluoreszenz. Die gestrichelte Linie zeigt die Grenze der Hintergrundfluoreszenz.

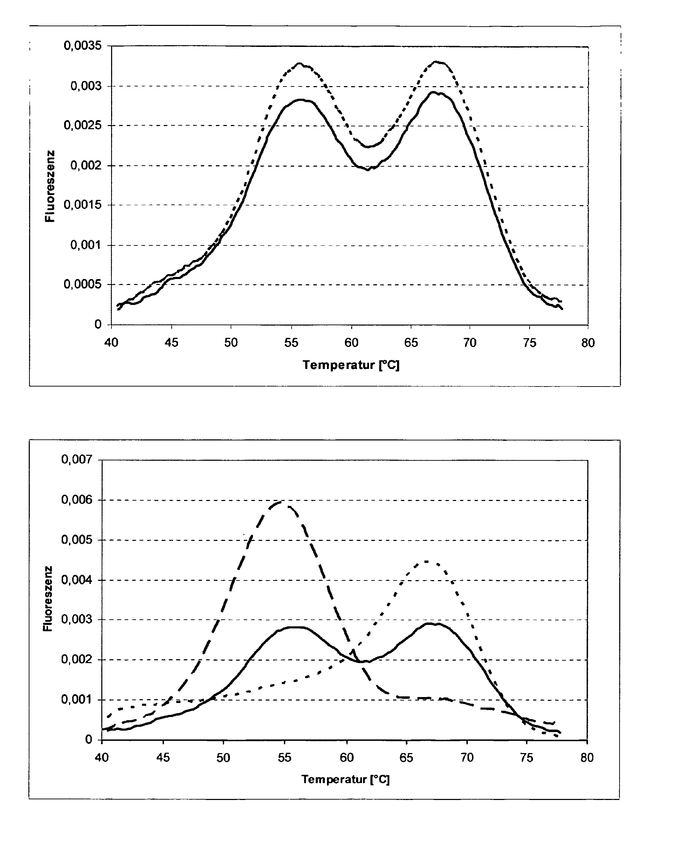

Abbildung 2 zeigt auf der linken Seite die Schmelzkurvenanalyse nach der isothermalen Amplifikation in Anwesenheit von PCR-Amplifikaten sowohl von *C. glutamicum* als auch *E. coli.* Auf der x-Achse ist die Temperatur in °C, auf der y-Achse die relative Fluoreszenz angegeben. Auf der rechten Seite ist zusätzlich noch als Kontrolle die Schmelzkurvenanalyse gezeigt, wenn entweder nur PCR-Amplifikate von *C. glutamicum* oder *E. coli* in die isothermale Amplifikation eingesetzt wurden.

Beispiele:
Die nachfolgenden Beispiele dienen der Erläuterung der Erfindung, ohne dass diese auf die Ausführungsbeispiele beschränkt wird.

Primersequenzen:
eCG-FWD:
5'-GCTCCAGCCACCCAAAAC
eCG-REV:
5'-GGCTTCATCGACAGTCTGACGACCGACTCAACCACTAATGCGTCGTC

eCG-PRO:
5'-6FAM-GGCTTCATCGACAGTCTGAC-BHQ1
eCG-ctrl:
   5'-GTCAGACTGTCGATGAAGCC
   EOF:
   5'-ATGCTACCCCTGAAAAACTC
   eEC-REV:
   5'-TTTACTTCTTTGCGTTATGTCTCTGACTCGCTTGAACTGATTTCCTC

eEC-PRO:
5'-6FAM-TTTACTTCTTTGCGTTAT-BHQ1

### Beispiel 1:

### Nachweis eines Teilbereichs der Nukleotidsequenz der Polyketid-Synthase von Corynebacterium glutamicum.

Mit Hilfe des Primerpaares eCG-FWD und eCG-REV (Sequenzen siehe oben), jeweils 2 pmol, wurde aus 10 bis 40 ng genomischer DNA aus *Corynebacterium glutamicum* in einer PCR ein Teilbereich der Nukleotidsequenz der Polyketid-Synthase amplifiziert. Der Reaktionsansatz besaß ein Volumen von 20 µl, zur Amplifikation wurde die HotStar-Taq DNA-Polymerase (QIAGEN) verwendet. Die Reaktionsbedingungen waren folgende:
1) 15 min 95 °C
2) 35 Zyklen:
   15 sec 55 °C
   40 sec 72 °C
   15 sec 94 °C
3) 2 min 72 °C
4) 5 min 98 °C

Nach der PCR-Amplifikation wurden 5 µl des Amplifikates der Zielnukleinsäure in die Reaktion zur isothermalen Amplifikation des Sequenz-Tags (Gesamtvolumen 10 µl) eingesetzt. In diese Reaktion wurden zusätzlich noch Desoxyribonukleotide (je 5 mM), 3 U N.BstNBI (NEB), 0,2 U Vent exo- (NEB) sowie 2 pmol des Oligonukleotids eCG-PRO gegeben. Der Reaktionsansatz wurde auf 56 °C erhitzt und die Temperatur während der gesamten Reaktionszeit (45 min) konstant bei dieser Temperatur gehalten. Alle 30 Sekunden wurde die Fluoreszenz gemessen.

Abbildung 1 zeigt den Anstieg der Fluoreszenz in Echtzeit. So ist auf dieser Abbildung zu erkennen, dass das Fluoreszenzsignal sehr schnell, nach 90 Sekunden, oberhalb des Fluoreszenzrauschens liegt und der Sequenz-Tag somit bereits nach 90 Sekunden der isothermalen Amplifikation nachweisbar ist. Die Fluoreszenzkurve erreicht nach etwa 40 Minuten ein Plateau, so dass nach dieser Zeit keine weiteren Sequenz-Tags mehr zusätzlich detektiert werden. Der Nachweis einer Zielnukleinsäure ist demzufolge mit dem erfindungsgemäßen Verfahren schnell und effizient möglich. Dieser Nachweis erfolgt sehr rasch, innerhalb von drei Minuten sind deutlich über dem Hintergrund liegende Signale zu detektieren.

### Beispiel 2:

Gleichzeitiger Nachweis eines Teilbereichs der Nukleotidsequenz der Polyketid-Synthase von *Corynebacterium glutamicum* und eines Teilbereichs der Nukleotidsequenz von Intimin von *Escherichia coli.*

Die Amplifikation der Nukleotidsequenz der Polyketid-Synthase wurde durchgeführt wie in Beispiel 1 beschrieben, die Amplifikation der Nukleotidsequenz von Intimin wurde in einer getrennten PCR durchgeführt. Die Reaktionsparameter waren dieselben wie in Beispiel 1 beschrieben, nur dass im Fall der Amplifikation der Nukleotidsequenz von Intimin 10 bis 40 ng genomische DNA von *Escherichia coli* eingesetzt wurden und zur Amplifikation die Primer EOF und eEC-REV (Sequenzen siehe oben) verwendet wurden.

Nach der PCR-Amplifikation wurden jeweils 2,5 µl der Amplifikate in einem Reaktionsgefäß in die Reaktion zur isothermalen Amplifikation der beiden Sequenz-Tags für 45 min eingesetzt. Die Reaktionsparameter waren dieselben wie in Beispiel 1 angegeben, nur dass zusätzlich noch 2 pmol des Oligonukleotids eEC-PRO (Sequenz siehe oben) in die Reaktion eingesetzt wurden.

Die Schmelzkurve wurde im Anschluss an die isothermale Amplifikation im Real-Time Cycler (MJ Research Opticon, Bio-Rad) durchgeführt. Abbildung 2 zeigt die Schmelzkurve der Sequenz-Tags mit ihren jeweils gegen sie gerichteten Sonden. Da sich die Schmelztemperatur der beiden Sequenz-Tags mit ihren jeweils gegen sie gerichteten Sonden unterscheidet, lassen sich durch eine Schmelzkurvenanalyse beide Sequenz-Tags gleichzeitig nachweisen. Die Schmelzkurve in Abbildung 2 (links) zeigt deutlich zwei Peaks, einen bei etwa 56 °C, den zweiten bei etwa 68 °C, was die Anwesenheit beider Sequenz-Tags in der isothermalen Amplifikationsreaktion beweist. Zur Kontrolle, Abbildung 2 (rechts), wurden zusätzlich die Schmelzkurven nach der isothermalen Amplifikation untersucht, wenn nur das Amplifikat der PCR von *Corynebacterium glutamicum* bzw. nur das Amplifikat der PCR von *Escherichia coli* in die isothermale Amplifikation eingesetzt wurde.

Somit konnte gezeigt werden, dass sich das erfindungsgemäße Verfahren auch hervorragend für Multiplex-Nachweise eignet.

## Patentansprüche

1. Verfahren zum Nachweis von Zielnukleinsäuren, umfassend die folgenden Verfahrensschritte:
a) Primer-vermittelte Amplifikation mindestens einer Zielnukleinsäure, wobei mindestens einer der zur Amplifikation verwendeten Primer ein Sequenz-Tag-Primer ist, der an seinem 5'-Ende einen nicht hybridisierenden Teil aufweist, wobei dieser nicht hybridisierende Teil eine erste Sequenz aufweist, die auf dem zu ihm komplementären neu synthetisierten Strang eine Schnittstelle für eine Nicking-Endonuklease während der Amplifikation erzeugt, und des Weiteren 5' von dieser ersten Sequenz eine zweite Sequenz aufweist, die auf dem zu ihm komplementären neu synthetisierten Strang einen Sequenz-Tag während der Amplifikation erzeugt;
b) in Kontakt bringen des zumindest teilweise doppelsträngigen Amplifikats aus Schritt a) mit Nukleotiden, einer Nicking-Endonuklease und einer Polymerase, wobei die Polymerase eine Strang-Verdrängungs-Aktivität und keine 5'→3' Exonuklease-Aktivität besitzt;
c) Isothermale Amplifikation des in Schritt a) erzeugten Sequenz-Tags durch eine oder mehrfache Wiederholung eines Zyklus, aufweisend die folgenden Schritte:
i) Einfügen eines Nicks an der in Schritt a) eingefügten Schnittstelle mittels der Endonuklease aus Schritt b);
ii) Auffüllen des Nicks beginnend an dem in Schritt i) erzeugten freien 3'-Ende mit komplementären Nukleotiden mittels der Polymerase aus Schritt b) unter gleichzeitiger Verdrängung des Sequenz-Tags aus dem Doppelstrang.
d) Spezifische Detektion des in Schritt c) amplifizierten Sequenz-Tags.

2. Verfahren gemäß Anspruch 1, wobei in Schritt a) zwei oder mehr Zielnukleinsäuren amplifiziert werden.

3. Verfahren gemäß Anspruch 2, wobei für jede der Zielnukleinsäuren in Schritt a) jeweils mindestens ein spezifischer Sequenz-Tag-Primer verwendet wird.

4. Verfahren gemäß einem der Ansprüche 1 bis 3, wobei die Amplifikation der Zielnukleinsäure aus Schritt a) mittels PCR erfolgt.

5. Verfahren gemäß einem der Ansprüche 1 bis 3, wobei die Amplifikation der Zielnukleinsäure aus Schritt a) mittels isothermaler Amplifikation erfolgt.

6. Verfahren gemäß einem der Ansprüche 1 bis 5, wobei die Nicking-Endonuklease aus Schritt b) aus der Gruppe bestehend aus Nt.BstNBI, Nt.BspQI, Nb.BbvCI, Nb.Bsml, Nb.BsrDI, Nb.BtsI, Nt.AlwI, Nt.BbvCI, Nt.CviPII, Nt.BsmAI, Nb.Bpu10I und Nt.Bpu10I oder einem Gemisch hiervon ausgewählt ist.

7. Verfahren gemäß einem der Ansprüche 1 bis 6, wobei die Polymerase aus Schritt b) aus der Gruppe bestehend aus Vent exo⁻, Deep Vent exo⁻, Bst exo⁻, dem Klenow Fragment der DNA Polymerase I, Phi29 DNA Polymerase, 9°Nm DNA Polymerase oder einer Mischung daraus ausgewählt ist.

8. Verfahren gemäß einem der Ansprüche 1 bis 7, wobei die Detektion aus Schritt d) mittels einer gegen den Sequenz-Tag gerichteten Sonde erfolgt.

9. Verfahren gemäß Anspruch 8, wobei die Sonde Fluoreszenz-markiert ist.

10. Verfahren gemäß einem der Ansprüche 8 oder 9, wobei die Detektion mittels Schmelzkurven-Analyse erfolgt.

11. Verfahren gemäß Anspruch 9, wobei die Fluoreszenz-markierte Sonde eine Dual-Labeled Probe ist.

12. Verfahren gemäß einem der Ansprüche 1 bis 11, wobei die Zielnukleinsäure genomische DNA, Plasmid-DNA, virale DNA, mitochondriale DNA oder plastidäre DNA oder ein Fragment einer oder mehrerer von diesen ist.

13. Verfahren gemäß einem der Ansprüche 1 bis 12, wobei die Polymerase eine DNA-Polymerase ist und es sich bei den Nukleotiden um Desoxyribonukleotide handelt.

14. Verfahren gemäß einem der Ansprüche 1 bis 13, wobei die Amplifikation aus Schritt a) mittels Nested-Primem erfolgt und der Sequenz-Tag-Primer ein Primer des inneren PrimerPaares ist.


SEQUENCE LISTING

<110>  QIAGEN GmbH

<120>  Verfahren zum Nachweis von Zielnukleinsäuren

<130>  PA436-EP

<140>  EP09010757.4
<141>  2009-08-21

<160>  7

<170>  PatentIn version 3.3

<210>  1
<211>  18
<212>  DNA
<213>  Corynebacterium glutamicum

<400>  1
gctccagcca cccaaaac                                                    18


<210>  2
<211>  47
<212>  DNA
<213>  Corynebacterium glutamicum

<400>  2
ggcttcatcg acagtctgac gaccgactca accactaatg cgtcgtc                    47


<210>  3
<211>  20
<212>  DNA
<213>  Artificial

<220>
<223>  DNA oligonucleotide with 6-FAM as fluorescence dye at its 5' end
       and BHQ1 as quencher at its 3' end

<400>  3
ggcttcatcg acagtctgac                                                  20


<210>  4
<211>  20
<212>  DNA
<213>  Corynebacterium glutamicum

<400>  4
gtcagactgt cgatgaagcc                                                  20


<210>  5
<211>  20
<212>  DNA
<213>  Escherichia coli

<400>  5
atgctacccc tgaaaaactc                                                  20


<210>  6
<211>  47
<212>  DNA
<213>  Escherichia coli

<400>  6

tttacttctt tgcgttatgt ctctgactcg cttgaactga tttcctc                47

<210>  7
<211>  18
<212>  DNA
<213>  Artificial

<220>
<223>  DNA oligonucleotide with 6-FAM as fluorescence dye at its 5' end
       and BHQ1 as quencher at its 3' end

<400>  7
tttacttctt tgcgttat                                                18